# EUROPÄISCHE PATENTSCHRIFT

(11) **EP 0 797 445 B1**
(45) Veröffentlichungstag und Bekanntmachung des Hinweises auf die Patenterteilung: **11.09.2002**
(21) Anmeldenummer: 95939285.3
(22) Anmeldetag: 15.11.1995
(51) Int. Cl.: A61K 33/22, A61K 31/695, A61K 35/78, A61K 31/69, A61K 33/00, A61K 33/34

(54) **VERWENDUNG VON BIO-ASSIMILIERBAREN WÄSSRIGEN BORVERBINDUNGEN ZUR BEKÄMPFUNG SUBVIRÖSER KRANKHEITSERREGER**
USE OF BIOASSIMILABLE AQUEOUS BORON COMPOUNDS FOR THE CONTROL OF SUBVIRAL PATHOGENIC AGENTS
UTILISATION DE COMPOSES AQUEUSES, BIOASSIMILABLES DE BORE POUR LA LUTTE CONTRE DES AGENTS PATHOGENES

(30) Priorität: 22.11.1994 DE 4441483
(43) Veröffentlichungstag der Anmeldung: 01.10.1997
(73) Patentinhaber: GSF - Forschungszentrum für Umwelt und Gesundheit GmbH, 85764 Oberschleissheim (DE)
(72) Erfinder: BENGSCH, Eberhard, D-81549 München (DE); KETTRUP, Antonius, D-59821 Arnsberg (DE); POLSTER, Jürgen, D-85356 Freising (DE)
(86) Internationale Anmeldenummer: EP9504494
(87) Internationale Veröffentlichungsnummer: WO96015798

(56) Entgegenhaltungen:
- EP-A- 0 354 522
- WO-A-92/01455
- FR-A- 2 684 002
- US-A- 4 285 934
- US-A- 4 963 655
- DATABASE WPI Week 9529 Derwent Publications Ltd., London, GB; AN 95-215940 XP002003184 & CN,A,1 086 662 (YANG) , 18.Mai 1994
- ACTA VIROL., Bd. 34, Nr. 4, 1990, Seiten 330-338, XP002003181 C.X. GEORGE ET AL.: "Studies on the antiviral activity of inorganic heteropolyanions against Semliki forest virus in vivo and vaccinia virus in vitro"
- LATV. PSR ZINAT. AKAD. VESTIS, KIM. SER., Nr. 6, 1985, Seiten 695-702, XP002003182 TERAUDA ET AL.: "Boric acid complexes with 5-hydroxysalicylic (gentisic) acid and alkali metals with antiviral activity"
- J. ORG. CHEM., Bd. 50, Nr. 6, 1985, Seiten 841-847, XP002003183 R.F. SCHINAZI ET AL.: "Synthesis of 5-(Dihydroxybory)-2'-deoxyuridine and related boron-containing pyrimidines"

## Beschreibung

Die Erfindung betrifft die Verwendung von wäßrigen lösungen von Borverbindungen zur Herstellung eines Arzneimittels zur Bekämpfung subviröse Partikel, die im Tier- und Humanbereich subakute, degenerative, nicht inflammatorische Erkrankungen des Zentralnervensystems, mit Ausnahme von Krankheit vom Alzheimer Typ, hervorrufen oder zum Schutz gegen die Wirkung subviröser krankheitserreger in Pflanzen.

Viroide sind infektiöse Partikel, die sich in adäquaten Wirtszellen autonom replizieren. Sie stellen die kleinste Form des Lebens dar. Mit subviralen Abmessungen von ca. 10 x 1 Nanometern sind die stäbchenförmigen Gebilde kleiner als das primitivste Virus. Sie bestehen aus einem ringförmigen Molekül Ribonukleinsäure: 246 bis 375 Nukleotide sind einzelsträngig und kovalent geschlossen zu einer Tertiärstruktur von hoher Stabilität vereinigt, wobei 2/3 der gegenüberliegenden Basen komplementär gepaart sind. Auch offenkettige, einfache und multimere Formen kommen vor. Viroide sind nicht von einer eigenen viroidspezifischen Proteinhülle umgeben. Viroide konnten als Partikel bis jetzt nur aus den Zellen höherer Pflanzen isoliert werden, jedoch wurden infektiöse Ribonukleinsäuren gleicher Art bei bestimmten Krankheiten auch in Vertebratenzellen nachgewiesen.

Neben der Abwesenheit zellularer Strukturen, einer Eigenschaft, die sie mit den Viren teilen, zeichnen sich diese kleinsten Parasiten dadurch aus, daß sie keine Proteine kodieren. Sie hinterlassen in der Proteinmaschinerie der invadierten Zelle keinen Fingerabdruck und entgehen auf diese Weise zumindest während der Inkubation den antiviralen Abwehrmechanismen des infizierten Wirtsorganismus.

In Pflanzen verursachen Viroide keine aktive oder passive Resistenz. Sie induzieren weder hyperergische Reaktionen, noch die Produktion von Antivirus-Proteinen.

Die Inkubationszeiten sind um Größenordnungen länger als bei Virusinfektionen.

Sie können bei langlebigen Pflanzen viele Jahre betragen. Jedoch bewirken hohe Temperaturen (tropisches Klima) eine drastische Verkürzung der latenten Perioden.

Die häufigsten Symptome einer Viroidinfektion in Pflanzen sind
- Wachstumshemmungen
- Stauchungen
- Nekrosen
- Kleinfrüchtigkeit und
- Absterben der Pflanze.

Die Symptome sind für alle Pflanzenarten und für alle Viroide qualitativ ungefähr gleich, was auf die Störung an zentralen Stellen der zellulären Regulation des Wirtes hinweist.

In Säugetiere injiziert verursachen Viroide keine Immunantwort, im Gegensatz zur Gesamtheit der Viren, einschließlich Pflanzenviren. Sie induzieren keine Interferonproduktion. Es kommt zu keiner serologischen Reaktion. Die Pathogenität der Viroide liegt jenseits der klassischen Definition der Infektionskrankheiten von Pasteur und Koch.

Die immunogenen Unterschiede zwischen beiden Erregerarten hängen damit zusammen, daß Viren transkriptionelle Krankheiten verursachen, Viroide hingegen post-transkriptionelle.

Viren entregeln primär die Synthese zellularer Proteine und kodieren an ihrer Stelle eigene Virusproteine, was im Säugetierorganismus zu einer sofortigen Immunantwort führt. Durch ihre relative Größe als Partikel und durch ihre Proteinund/oder Glykoproteinumhüllung sind klassische Viren das Modell für ein potentielles Antigen. Virale Proteine werden auf den Histakompatibilitäts-Molekülen der Klasse I (MHC I) präsentiert.

Viroide und viroidanaloge Krankheitserreger benutzen hingegen ausschließlich die Proteine des Wirtes. Dies und ihre Kleinheit schließen von vornherein jede Präsentation als Antigen und die nachfolgende immunogene Reaktion aus.

Die metabolische Schadwirkung der Viroide beginnt relativ spät durch post-transkriptionelle Störung des wirtseigenen Proteinund Glykoproteinzyklus. Unter der Wirkung der Viroide kommt es bei ihnen zu:
- Funktionsentfremdung
- Behinderung des transmembranären Transportes
- Blockierung des Abbaus
- Proteinpolymorphismus als Folge fehlerhafter Prozessierung.

Dies führt zu konzentrationären Ansammlungen von nichttransferierten, nicht rezyklierten und ggf. prozessierten Wirtsproteinen in stagnierenden zellulären oder interzellulären Pools, wo sie unter Kristallisierung, Mineralisierung, Fibrillisierung und/oder Amyloid-Plaques-Bildung für Proteinasen unangreifbar werden und irreversibel zur Ablagerung gelangen. Die viroidbedingte Fehlfunktion der körpereigenen Proteine erlaubt es dem Immunsystem lediglich, Auto-Immun-Antikörper einzusetzen und dies in einer späteren Phase, nachdem der Störfall irreversibel geworden ist, d. h. im Endstadium der Krankheit. Tatsächlich werden in den späten letalen Stadien von durch viroid-ähnliche Partikel verursachten subakuten Erkrankungen bei Säugetieren Auto-Immun-Antikörper aufgefunden, die gegen die kristallisierten wirtseigenen Proteine gerichtet sind. Bei Mäusen wurde ein Einfluß der Histakompatibilitätsgene der Klasse II (MHC II) auf den Infektionsverlauf festgestellt. Die schwammartige Durchlöcherung befallener Nervengewebe als Folge entzündungsfreier, spontaner Zellzerstörung erinnert an apoptotische Selbstauslöschungsmechanismen der Zellen, wie sie durch autoimmune Signalgebung ausgelöst werden können.

Ihre Primitivität und ihre sequenzielle, genomische Nähe sowie ihre Komplementarität zu funktionellen Wirtszellenribonukleinsäuren (Intronen, 7S-, 7SL-RNAs, Ribonukleasen P und L) hat Viroide und assimilierte infektiöse Partikel bis jetzt jeder selektiven kurativen Einflußnahme entzogen, weil jede gezielte Einwirkung auf den viroiden Parasiten essentielle Wirtszellenfunktionen in Mitleidenschaft zieht.

Von allen Parasiten ist bei Viroiden und anderen subvirösen Molekülen die funktionelle Nähe zu den normalen Bestandteilen der Wirtszelle am größten, was von vornherein maximale Schwierigkeiten bei ihrer Identifizierung und Bekämpfung erwarten läßt.

Viroide aus Pflanzenzellen sind als Einzelstruktur isolierbar und elektronenmikroskopisch sichtbar. Blot-Methoden sind hochempfindlich und erlauben es, noch ein Viroid pro Zelle nachzuweisen.

Im animalen und im Humanbereich gelang es hingegen bisher nicht, krankheitsspezifische, viroidanaloge Ribonukleinsäuren, die in infektiösen Geweben aufgefunden wurden, in freier Form als unverpackte, autonom operierende Partikel zu sichten und zu isolieren. Unter den physiologischen Bedingungen der Tierzelle werden Ribonucleinsäuren noch während der Transkription, der Replikation und dem "Processing" an dem sich bildenden Strang proteinverpackt. Spezifische Bindungsaffinitäten zu Molekülen der Wirtszelle widersetzen sich der Existenz nackter RNA-Viroidpartikel. Zudem begünstigen die Körpertemperaturen, die in Warmblüterwirten herrschen, die Auflösung eventuell möglicher autonomer Strukturen tierischer Viroide. Bei 38° C liegen auch Pflanzenviroide bereits in geschmolzenen, anlagerungsaktiven Modifikationen vor.

Die bei intermolekularen Assoziationen auftretenden Energien sind größer als die, die durch die Betätigung der noch verfügbaren intramolekularen, selbstkomplementären Basenpaarungen gewonnen werden können (1,2 kcal/Nukleotidpaar). Die Komplexe sind stabiler als das Viroidpartikel in sich selbst. Viroidähnliche Erreger in tierischen Zellen befinden sich permanent im ekliptischen Zustand. Ihre Übertragung mit Hilfe des infizierten Zellmaterials erfordert nicht die Bildung einzelverpackter, sichtbarer Partikel wie bei den Viren, was bereits an der niedrigen Codier-Kapazität solcher viroider und anderer subviröser RNAs scheitern würde.

Spezifische intermolekulare Bindungen subviröser RNAs existieren zu wirtszellulären Nukleinsäuren (anti-sens-RNA-Segmente, anti-genomische Bindung), zu Lipiden (Phospholipide) und zu den enzymatisch und physiko-chemisch hochresistenten, fibrillären oder amyloiden pathogenen Protein-Assoziaten. Die Erreger sind verpackt und maskiert. Das Assoziat wird durch gegenseitige Stabilisierung seiner Komponenten biologisch unzerstörbar.

Fibrilläre und amyloide Protein-Aggregate bilden sich in stagnierenden Pools, unter mutmaßlicher Mitwirkung der viroiden Parasiten. Dabei kommt es u. a. zu post-transkriptionellen Punktmutationen von wirts-kodierten, normalerweise nicht pathogenen Proteinen: An bestimmten Positionen werden hydrophile Aminosäuren durch lipophile und saure durch basische Aminosäuren substituiert. Den gebündelten, pathogenen, hochresistenten Prion-Proteinaggregaten werden von einigen Autoren sogar autoreplikative Eigenschaften zugeschrieben.

Parallel dazu steigt bei den befallenen Neuronen die Affinität zu basischen mikroskopischen Färbemitteln (basophiler Charakter).

Eine starke lipophile Affinität der Erreger oder ihrer Assoziate trägt dazu bei, Abwehrreaktionen aus dem wäßrig-serologischen Milieu zu unterlaufen. Das Ausweichen auf das lipophile Milieu äußert sich auch in der Präferenz der Erreger für lipidreiche Gewebe im invadierten Wirt: Hirn, Nervenbahnen, Spinalstrang, Hypophyse, Milz, Augen, Lymphocyten, Darmtrakt und alle sekretorischen Gewebe sind die Hauptzielorgane.

Erreger in solchen infektiösen Geweben können durch Behandlung mit lipophilen Lösungsmitteln (Aceton) inaktiviert werden. Plaques und Fibrillen der Prion-Proteine können durch aufeinanderfolgendes Einwirken von Nukleasen und Proteinase K zur Auflösung gebracht werden.

Während der Replikationszyklus der Viroide in Pflanzenzellen aufgeklärt ist, ist die Aktivität viroid-ähnlicher Partikel im Tier- und Humanbereich noch weitgehend unverstanden.

Bekannt hingegen sind die hohe Infektiosität aller Viroidarten und die Quasi-Unzerstörbarkeit der viroiden Partikel. Im Gegensatz zu Bakterien und Viren sind sie aus einem kontaminierten Lebensraum praktisch nicht mehr zu vertreiben und führen zu einer kumulativen Biokontamination.

Daraus resultiert die erhebliche wirtschaftliche Bedeutung, die einer zerstörungsfreien Beherrschung von Viroidinfektionen im Pflanzen- und Tierbereich zuzumessen ist.

In der Pflanzenwelt führen Viroidinfektionen zu enormen, großflächigen Schäden, vor allem in tropischen Klimazonen. Dort werden Wild- und Kulturpflanzen in gleicher Weise betroffen.

Dem Coconut Cadang Cadang Viroid (CCCVd) sind bereits 30 Millionen Kokospalmen (Cocus nucifera) zum Opfer gefallen. Die ultra-kleinen Partikel von 10 nm bringen 20 m hohe Baumriesen zum Absterben, auf den Philipinen 800 000 pro Jahr. Die Zerstörung in den sich herdartig ausbreitenden Zonen ist so gründlich und die Transmission der Viroide so unvermeidlich und prompt, daß eine Auslöschung der betroffenen Art und der verwandten viroid-toleranten Spezies befürchtet werden muß.

In gemäßigten Klimagebieten betreffen schwere Viroidepidemien selektiv Monokultur- und Gewächshauspflanzen, dabei vorzugsweise die auf bestimmte Eigenschaften gezüchteten Hochleistungssorten. Wildpflanzen entwickeln kaum Symptome, stellen aber ein permanentes Reservoir für neue Epidemien in den Kulturpflanzen dar.

Eines der virulentesten Viroide in unserer Klimazone ist das hier beschriebene Spindelknollenviroid der Kartoffeln (Potato Spindle Tuber Viroid = PSTVd). Es ist polyvalent und befällt eine große Zahl von dikotylen Kulturpflanzen. Mit 356 bis 360 Ribonukleotiden gehört es zu den großen Viroiden. (Die obere Existenzgrenze liegt für die bisher bekannten Viroide bei 375 Ribonukleotiden).

Große Viroide wie PSTVd haben einen weiten Wirtskreis. Kleine Viroide, wie das oben erwähnte CCCVd (246 Nukleotide), befallen nur eine oder wenige eng miteinander verwandte Arten. Dabei ist zu bemerken, daß die bekannten Viroide den schmalen Existenzraum zwischen 246 und 375 Nukleotiden nicht kontinuierlich, sondern gequantelt ausfüllen: An der Ober- und Untergrenze und ungefähr alle 20 Nukleotide häufen sich die verschiedenen Viroidarten; die übrigen Nukleotidzahlen bleiben unbesetzt.

PSTVd kann in Isolaten mit milder, strenger oder letaler Symptomausprägung auftreten. Mit strengen Isolaten infizierte Feldkartoffelkulturen erleiden in warmen Sommern Ertragseinbußen von 60 % (Wachstumsverzögerung, Kleinfrüchtigkeit). Gewächshauspflanzen sind noch anfälliger. Ein virulenter PSTVd-Stamm kann Treibhauskulturen zu 100 % auslöschen.

Im tierischen und im Humanbereich werden viroid-ähnliche Partikel und in virale Genome integrierte Viroid-Segmente mit einer Reihe degenerativer, nicht- oder postinflammatorischer Erkrankungen des Zentralnervensystems, mit Ausnahme von krankheiten von zheimer Typ, in Verbindung gebracht. Sie können nach langen Inkubationszeiten und Latenzperioden zu schweren neuropathologischen Störungen führen und verlaufen von da ab stets tödlich.

Viroidähnliche Parasiten werden in Säugetieren und im Menschen ursächlich oder als Ko-Faktor mit ungefähr 50 subakut-chronischen Erkrankungen in Verbindung gebracht. In den folgenden 5 Gruppen werden diese nach sinkender Absicherung der Kenntnisse über die Viroidbeteiligung geordnet:
**1. Virosoide** sind definierte, z. T. sequenzierte Partikel, die eine nichtkodierende Viroidkomponente enthalten und mit Hilfe eines Helfervirus infektiös werden. Sie treten in Pflanzen (Satellitenviren, multipartite virale Genome) und Tieren auf.
   Im Hepatitis Delta Virosoid (HDV) hat sich ein 370 Ribonukleotide langes Viroid mit einer 4 x so großen Satelliten-RNA zusammengetan und verursacht im Menschen als Ko-Infektant von Hepatitis B schwere hepatische und neuro-pathologische Komplikationen.
**2. Nichtkonventionelle Lenti-Viren** stellen eine weitreichende, noch wenig definierte Gruppe dar. Dazu gehören die Erreger degenerativer, nach Ausbruch der klinischen Symptome stets tödlich verlaufender Erkrankungen des Zentralnervensystems mit den oben erwähnten pathologischen und histochemischen Effekten: schwammartige Durchlöcherung von Hirn- und Nervengewebe durch das Absterben von Nervenzellen, Bildung fibrillärer und amyloider Proteinaggregate durch fehlerhaftes Prozessing von Genprodukten, Astrocytose (Hypertrophie der Astrocyten unter Bildung saurer, fibrillärer Glykoproteine), Verhaltensstörungen, progressive Pathogenese zahlreicher anderer Organe.
   Zu den spongiformen Encephalopathien gehören: Die Scrapie-Erkrankung von Schafen (z. B. 18 000 Fälle bei einem Impfunfall in Großbritannien), der Rinderwahnsinn (Bovine Spongiforme Encephalitis = BSE, 10 000 Fälle jährlich in Großbritannien) und beim Menschen die Creutzfeldt-Jakob-Erkrankung (CJE), das Gerstmann-Sträußler-Syndrom (GSS), die Kuru- und die Alpers'sche Krankheit. Die Erreger überwinden,die Artenbarriere und sind für alle Säugetiere potentiell infektiös.
   Die Bedeutung der nichtkonventionellen Viren erhöht sich durch den Verdacht ihrer Mitwirkung an einer langen Liste anderer chronischer Erkrankungen: Alzheimersche Demenz (klinisch nicht unterscheidbar von einer langsamen CJE, 800 000 potentielle Fälle in Deutschland, in immer jüngerem Alter auftretend), die Parkinsonsche Krankheit, zahlreiche Auto-Immunkrankheiten, Sklerosen, z. B. die laterale Sklerose, Epilepsien, Schizophrenien, endogene Depressionen, Verhaltensanomalien und der Autismus.
**3. Homologien der Viroide mit Retroviren und zelltransformierenden DNA-Viren** Retroviren und Viroide weisen eine Reihe von Gemeinsamkeiten auf: Klassische Viren verursachen inflammatorische, Viroide degenerative Prozesse. Retroviren vereinigen beide Eigenschaften und stehen funktionsmäßig als Brückenglied zwischen den klassischen Viren und Viroiden.
   Viroide besitzen ihrerseits retrovirale Eigenschaften. Durch ihre Fähigkeit, sich in ein Genom zu integrieren und wieder auszuspleißen,sind sie auf RNA-Ebene das funktionelle Äquivalent der retroviralen Proviren im DNA-Genom, mit deren "long terminal repeats" (LTR) Sequenzhomologien bestehen. Solche nicht-kodierenden Sequenzwiederholungen spielen bei der Übersetzung und Rückübersetzung viraler Informationen zwischen RNA und DNA eine Rolle. Hochkonservierte Viroid-Sequenzen, die in allen Viroiden vorkommen, scheinen auch in retroviralen Genomen aktiv zu sein.
   Auch für den Replikationszyklus bestehen Analogien:
   Retroviren und Viroide benutzen die gleichen zellulären Enzyme: DNA-abhängige RNA-Polymerasen.
   Eine gezielte Modifikation der pathogenen Eigenschaften der relativ einfachen Viroide kann zum besseren Verständnis der Rolle der LTR in den sehr viel komplexeren Retroviren und einem regulativen Einfluß auf die letzteren beitragen.
   Darüber hinaus besitzen die Genome der DNA Tumorviren aus den Familien Parvo, Hepadna, Polyoma, Adeno und Herpeto ebenfalls Sequenzwiederholungen, die typische konservierte Viroidsequenzen als Grundbaustein enthalten. Mit ihrem vielfach wiederholbaren "Rolling Circle"-Mechanismus stellen Viroide das Urmodell für die Entstehung und Funktion nicht-kodierender Sequenzwiederholungen auf RNA- und DNA-Ebene dar.
   Ganz allgemein scheinen konservierte Viroidsequenzen (z. B. die zentralen palindromischen Dekanukleotide der Viroide) für das Wechselspiel der Viren zwischen der aktiven Replikationsphase und dem zeitweiligen Untertauchen im DNA-Genom eines während dieser Periode symptomfreien Wirtes notwenig zu sein.
**4. Subakut persistierende klassische Viren**
   Eine Reihe von degenerativen Erkrankungen wird von klassischen RNA-Viren verursacht. Nach Abklingen der Hauptinfektion überleben virale Subspezies in defektivem Zustand mit verändertem Antigenmuster die Immunabwehr, persistieren in den Nervenbahnen und kriechen dort, ähnlich den Lyssaviren (Tollwut), von Zelle zu Zelle. Nach z. T. sehr langen Latenzzeiten kommt es zu schweren, meist tödlich verlaufenden, neuropathologischen Spätfolgeerkrankungen (Masern-, Rubella und Polioviren oder bei der Multiplen Sklerose als Spätfolgeinfektion nicht-permissiver Humanzellen durch Theilers Murines Poliovirus).
   Eine viroide Mitbeteiligung an solchen Spätfolge-Infektionen wird aus zwei Gründen diskutiert:
   Viroide stellen hochkomplementäre Doppelstrang-RNA dar. Solche Segmente dienen einerseits als Induktionssignal für die Interferonproduktion, die über die mehr oder weniger vollständige Neutralisierung konventioneller Viren während der akuten Initialinfektion entscheidet. Funktioniert dieser Mechanismus unvollständig, bleiben klassische Viren als potentielle Verursacher von Spätfolgeinfektionen übrig.
   Andererseits funktionieren Doppelstrang-RNA-Segmente als Drehscheibe zur Stabilisierung von RNA-Virusgenomen und entscheiden auf diese Weise über die Streubreite der Quasi-Spezies-Populationen und die Menge der gebildeten defektiven Sub-Spezies. Je weiter solche defektiven Unterarten von dem initialen Antigenmuster entfernt sind, desto höher ist ihre Überlebensrate.
**5. Viroidbeteiligung an genetischen Krankheiten.**
   Die Eizelle ist im Pflanzenbereich Hauptziel und wichtigster Vektor der Viroidinfektion. In Analogie dazu sind auch im Tierbereich vertikale Viroidübertragung mit chromosomalen Schädigungen an der Eizelle nicht auszuschließen. Bei genetischen Krankheiten, die vom mütterlichen Teil abhängen und dabei mit dessen Konzeptionsalter korrelieren (z. B. Down-Syndrom), ist eine Viroidbeteiligung an der Auslösung der Chromosomenanomalie denkbar. Ein Hinweis dafür ist auch die Hirnamyloidose, die im Trisomie-Krankheitsverlauf auftreten kann und sonst für nichtkonventionelle, atypische "Slow-Virus"-Erkrankungen charakteristisch ist.
   Viroide und assimilierte infektiöse Partikel zeichnen sich durch eine außergewöhnliche physikalische, chemische und biologische Resistenz aus.
   Die Inaktivierung durch UV-, ionisierende und Neutronenstrahlung, durch Hitze, Formaldehyd und Alkohol und durch Nukleasen und Proteinasen ist nur beschränkt wirksam.
   Das Potato Spindle Tuber Viroid ist 20 x widerstandsfähiger gegen UV-Bestrahlung als die stabilsten Viren, Scrapie-Agens sogar 200 x. Letzteres ist auch gegen Magensäure und gegen Temperaturen von 350° C resistent. Von scrapie-erkrankten Schafen benutztes Weideland ist noch nach Jahren infektiös.

Das Versagen der klassischen Dekontaminations- und Desinfektionsmethoden wurde im human- und tiermedizinischen Bereich an spektakulären Unfällen demonstriert.

Viroide aus erkrankten Kulturpflanzen lagern sich symptomlos als latente Viroide oder Spurenviroide in Wildpflanzen ein und warten dort auf adäquate Wirte. Sie etablieren sich in dem gegebenen biologischen Lebensraum definitiv und überdauern dessen Wandlungen.

Durch die industrielle Landwirtschaft mit dem großflächigen Einsatz von Hack- und Schneidegeräten wird für eine regelmäßige, massive Durchmischung aktiver und latenter Viroidreservate gesorgt. Die Vektordichte und -geschwindigkeit der Viroidübertragung sind hoch. Multiinfektionen (Viroide, Pflanzenviren) häufen sich. Für diese durch die Industrialisierung der Natur gegen die Evolution abrupt geschaffene Situation existieren keine adäquaten Abwehrmechanismen. Es kommt zur Großflächenzerstörung sensitiver (viroid-toleranter) vegetaler Arten und zu einer kumulativen Biokontamination der Pflanzenwelt durch immer mehr Viroidarten und -stämme.

Auch im Tierzucht- und Humanbereich häufen sich die Risikofaktoren für eine systematische Transfektion der Populationen durch die Erreger der subaktuen Encephalopathien, die mit viroidähnlichen RNA-Molekülen in Verbindung gebracht werden.

Die Massentierhaltung unter industriellen Bedingungen und die Rezyklierung von Schlachtresten zu Wiederverfütterungszwecken führten zu einer epidemischen Ausbreitung von Scrapie, Rinderwahnsinn BSE und deren Varianten unter allen Zucht- und Nutztieren. Unter wild lebenden Tieren der gleichen Art sind derartige Epidemien unbekannt, zumindest verlaufen sporadische Infektionen symptomfrei. Werden dieselben Tiere den Bedingungen der Tierhaltung oder des zoologischen Gartens unterworfen, erkranken sie ebenfalls.

Die verschiedenen Erreger überwinden mehr oder weniger leicht die Artenbarriere zwischen den Säugetierarten, ggf. über einen Zwischenwirt, in dem sie wie die Pflanzenviroide zunächst einmal symptomlos bleiben können. Serienpassagen im neuen Wirt verkürzen dann die Inkubationszeiten und erhöhen die Virulenz.

Die Verwendung seit 1981 von Futtermehl aus scrapie-erkrankten Schafen führte 1987 in England zum Ausbruch von Rinderwahnsinn (BSE), einer vormals unbekannten und heute in England epidemisch und weltweit sporadisch verbreiteten Krankheit. Alle Schlacht- und Nutztiere sind infizierbar. Industrielle Aufzuchtmethoden, Impf- und Dopingpistolen potentialisieren den Effekt.

Dabei scheint für die verschiedenen Erregerisolate eine Korrelation zu bestehen zwischen der Leichtigkeit, mit der sie die Artenbarriere überspringen und sich an den neuen Wirt adaptieren und der Gründlichkeit, mit der sie die betreffenden Individuen der befallenen Population ausrotten: Der mäßig adaptive, artenstabile Scrapie-Erreger der Schafe ist nur schwierig auf andere Tierarten zu übertragen, vernichtet aber seine Wirtsstammart, die befallene Schafherde, vollständig. Der leicht artenspringende, d. h. auf andere Tierarten übertragbare bovine BSE-Erreger hingegen führt nur bei wenigen Individuen einer befallenen Rinderherde zu letalen Symptomen. Das heißt, bei einem relativ unspezifischen Erregerstamm (breites Wirtsspektrum) besteht für das infizierte Individuum eine bessere Chance, die letale Symptomausprägung zu verzögern oder zu vermeiden.

Um die potentielle Gefahr im Humanbereich abzuschätzen, muß der Mensch ebenfalls als konzentrationär lebendes Säugetier betrachtet werden.

Eine Tier- zu -Mensch-Übertragung durch kontaminierte Schlacht- und Nutztiere ist am Beispiel Scrapie(Schaf)-Creutzfeldt-Jakob-Enzephalopathie (Mensch) epidemiologisch, in Richtung Mensch-Tier experimentell erwiesen. Als besonders infektiös erwiesen sich die oben beschriebenen Zielorgane der Erreger: Nerven, Lipid- und sekretorische Gewebe, deren Verkauf aus boviner und oviner Herkunft in Frankreich bereits gesetzlich verboten wurde. Aber auch Muskelgewebe und Milchprodukte sind aus der Kontaminationsreihe nicht auszuschließen.

Die Mensch-zu-Mensch-Übertragung der spongiformen Encephalopatien im medizinisch-pharmazeutischen Bereich ist durch dramatische Einzelfälle belegt. Das epidemische Ausmaß ist wegen des kürzlichen Auftretens der Erreger und der extrem langen Inkubationszeiten noch nicht abzuschätzen. Präventive Maßnahmen werden behindert durch
- die Quasi-Unzerstörbarkeit der Erreger (klassische Hygienemethoden durch Chemie, Hitze, Strahlung und Enzyme versagen)
- die Universalität der Übertragungswege (die Rolle der Übertragungsweise beschränkt sich auf die Beeinflussung der Dauer der symptomlosen Latenzperiode, z. B. wirken, wie bei Tollwut beschrieben, massive, hirnnahe, serale Inokulationen am schnellsten, während die perorale Aufnahme geringer Mengen infektiösen Materials zu extrem langen Inkubationszeiten führt.
- das Fehlen eines adäquaten Sicherheitstests (der Tod des Patienten ist das einzige Kriterium).

Eine postinfektionelle Behandlung war bisher ebenfalls nicht bekannt.

Das potentielle Risiko einer Übertragung besteht beim augenblicklichen Stand der Technik
- bei chirurgischen und dentalen Eingriffen (chirurgische Instrumente, Implantate, Elektroden), z.B. haben in dentalen Nerven angereicherte Erreger zur Kontamination von Zahnärzten und ihrer Klienten geführt.
- bei allen Organ-, Gewebe- und Knochenmarktransplantationen (besonders risikobeladen sind die nach dem Ableben des Spenders bereits angealterten, aber noch verwendbaren Organe, wie Hornhaut, Dura Mater; die Virulenzstimulation auf die Erreger wird dabei mit dem verstärkten Auftreten von Streßproteinen im absterbenden Spenderorganismus in Verbindung gebracht, d. h. je später das kontaminierte Gewebe im sterbenden Spender ablatiert wird, desto infektiöser wird es.
- bei Transfusionen und seralen Produkten (eine sichere Sterilisierung oder Reinigung der Produkte ist wegen der Kleinheit und Resistenz der Erreger nicht realisierbar)
- bei Extrakten aus menschlichen und tierischen Organen (Hormone, Interferone) z. B. führte die Verwendung von Wachstumshormonen aus Hypophysen verstorbener Spender zur Kontamination aller mit einer gegebenen Probe behandelten Kinder im Alter von 4 bis 16 Jahren.
- Risikobelastet sind ebenfalls alle pharmazeutischen Stärkungs- und Regenerierungspräparate, die Extrakte lipidreicher tierischer Organe enthalten.

So sind alle negativen Umstände für eine kumulative Bio-Kontamination sowohl der höheren Pflanzen durch Viroide als auch der höheren Tiere durch viroidähnliche Partikel gegeben.

Der Krieg der Kleinsten gegen die Größten wird mit Minimalunterschieden im Metabolismus ausgetragen. Die Informationsarmut und die Codier-Unfähigkeit der Erreger bewirken, daß diese ausschließlich mit Hilfe von Wirtsfaktoren proliferieren.

Bei ihrer Ausbreitung über Individuen und Arten wächst die Virulenz der Erreger (im Gegensatz zu Viren, wo der Artentransfert z. B. die Bildung abgeschwächter Virus-Impfstämme erlaubt).

Durch die Industrialisierung der Natur steigt die Anfälligkeit der Wirte: Hoch anfällig sind vor allem überzüchtete Tiere und Pflanzen in Massenhaltung und in Monokultur.

Dies führt dazu, daß sich Punktmutationen und statistisch seltene Fälle des fehlerhaften Prozessings von Gen-Produkten, die sporadisch an einem Individuum auftreten können, zu einer globalen Pandemie aufschaukeln können, während sie bei den unterkritischen Bedingungen der prä-industriellen Gesellschaft inselartig beschränkt blieben und in Selbstauslöschung endeten.

Ein Miniatur-Modell für den global-ökologischen Problemfall der "slow-virus"-Epidemien ist die Kuru-Krankheit auf Neuguinea, die mit Sicherheit auf einen sporadischen Fall von Creutzfeldt-Jakob (CJE) bei einem Papua-Ureinwohner zurückzuführen ist. Der rituelle Bestattungs-Kannibalismus wurde zum Vektor der Kuru-Epidemie, der auch unbeteiligte Kleinkinder zum Opfer fielen, und dies lediglich durch zufällige Kontaktkontamination mit dem infektiösen Gewebe. 1957 wurde der mortuäre Ritus gesetzlich unterbunden. Damit wurde der Hauptvektor hinfällig. Die Epidemie ging zurück. Vor allem erkrankten keine danach geborenen Kinder mehr. Jedoch kommt es noch heute unter den Papua-Erwachsenen in aller Welt zu Todesfällen, die auf eine Kontamination ehemaliger Ritual-Teilnehmer und ihrer Angehörigen vor mehr als 30 Jahren zurückgehen.

Alles in allem hat sich in wenigen Jahren eine Summe von Risikofaktoren herausgebildet: Gefährliche, infektiöse, hochresistente, ubiquitäre, ultrakleine Erreger mit extrem langen Inkubationszeiten und metabolischen Minimalunterschieden zu Wirtsfaktoren stehen immer anfälliger werdenden Wirtsorganismen gegenüber.

Im Tier- und Humanbereich fügen sich die Unentdeckbarkeit der Erreger, die Polyvalenz ihrer Übertragung, das Fehlen adäquater Testsysteme und die unspezifische Krankheitssymptomatik hinzu, die eine sichere Diagnose erst post mortem erlaubt.

Ein Gegenmittel muß in der Lage sein, auf Wirts- und Erregerseite hochspezifisch zwischen den normalen und den quasi-identischen pathogenen Molekülen zu unterscheiden und selektiv auf die letzteren hemmend einzuwirken.

Ein derartiges Gegenmittel gegen Viroide und viroidartige Erreger war bis jetzt, mit Ausnahme von Zellgiften, nicht bekannt.

Aufgabe der Erfindung ist es, eine neue Verwendung von bio-assimilierbaren Borverbindungen aufzuzeigen.

Gelöst wird diese Aufgabe durch die Merkmale des Patentanspruchs 1.

Die Unteransprüche beschreiben vorteilhafte Ausgestaltungen der Erfindung.

Vorteilhaft wirkt sich die erfindungsgemäße Verwendung von wäßrigen Lösungen von Borverbindungen zum Schutz von Pflanzen, Feld-, Garten- und Gewächshauskulturen, Baum- und Strauchbeständen gegen pathogene Viroidinfektionen aus, wobei den Pflanzen im Boden oder sonstigen Nährmedien und/oder auf jede andere Weise bioassimilierbare, anorganische oder organische Borverbindungen im Rahmen der für die Spezies und Sorte geltenden Toleranzgrenzen zugeführt werden und wobei die Applikationssform jede Art von boden-, pflanzen- und allgemein bioverträglichen anorganischen und organischen Borverbindungen oder Mischungen daraus sein können, vorzugsweise wäßrige Lösungen von Borsäure, von Boraten, die ggf. neutralisiert werden, von organischen Borverbindungen, insbesondere von Boratchelatkomplexen mit Polyolen wie Glykol, Glycerin, Mannit oder Sorbit, mit Oxycarbonsäuren, wie Milch-, Wein- oder Zitronensäure oder in Form von Kohlenhydratkomplexen mit Glukose, Galaktose, Fruktose, Maltose, Laktose oder jeder anderen Art von Mono-, Di-, Tri- und Oligosacchariden als glykosidische Liganden.

Die Applikation der borhaltigen Lösungen kann nutritiv über Böden oder in Hydrokultur oder folial durch Versprühen oder über beide Wege erfolgen, wobei für Böden neutralisierte Borate, für Hydrokulturen und bei folialem Versprühen Boratkomplexe mit Polyhydroxyverbindungen bevorzugte Anwendungsformen darstellen.

Bei der Tomatensorte Rentita stellen ein permanenter Gehalt von 1 ppm Bor in Nährlösung und in Torfbodenkultur ein regelmäßiges Begießen mit Lösungen von 2-5 ppm Bor in Mengen, die einer Gesamtborzufuhr von 2 bis 5 kg Bor/ha entspricht, vorteilhafte Dosierungen dar und führen zu einer 10 bis 15 fachen Boranreicherung (100 - 150 ppm) gegenüber unbehandelten Pflanzen (10 - 15 ppm Bor) bei gleichzeitig gesteigertem Wachstum.

Bei der Dosierung von Bor in Böden ist es vorteilhaft auch deren Adsorptions- und Desorptionseigenschaften für die entsprechenden Borverbindungen ggf. nach den klassischen Methoden vorzuermittelt und in Rechnung zusetzen.

Die obere Toleranzgrenze für Bor wird durch simultane Applikation von bioverfügbarem Silizium bei nochmals gesteigertem Pflanzenwachstum in beträchtlich höhere Konzentrationsbereiche verschoben, was bei Tomaten eine Boranreicherung um das 20 - 30-fache ihres natürlichen Borgehaltes bewirkt, wenn die Hydrokulturnährlösung beispielsweise 1 - 2 ppm B und 100 ppm Si in Form von neutralisiertem Wasserglas enthält.

Bei Verwendung großtechnisch verfügbarer Formen von löslichen Silikaten wie Hüttenschlacke kann die Wirkung anderer darin enthaltener Spurenkomponenten wie Mangan an dem System Pflanze/Boden/Viroid vorermittelt und berücksichtigt werden.

Nichtpathogene, inoffensive Viroidstämme, die durch Manipulation in hochborangereicherten Pflanzen gezüchtet werden, besitzen ein starkes Proliferationsvermögen, d. h. sie weisen hohe Infektiosität bei geringer oder ohne Toxizität auf.

Die Verwendung solcher nichtpathogenen, bormodifizierten Viroidstämme, z. B. in Form von infiziertem Zellmaterial, zur präventiven Beimpfung gefährdeter Kulturen als Infektionsschutz gegen verwandte Viroidstämme hoher Pathogenität führt durch Ausnutzung von Inkompatibilitätsphänomenen dazu, daß das erstinfizierende, zellbesetzende Viroid die Entwicklung nachfolgender, verwandter Viroide unterdrückt.

Bei Verwendung von Extrakten aller Art aus borangereicherten Pflanzen sollen vorzugsweise Pflanzenarten Verwendung finden, die bereits im natürlichen Milieu eine hohe Borakkumulation aufweisen können, wie z. B. Crocus sativus (bis 5000 ppm Bor) oder die Papaverarten (300 - 1000 ppm Bor).

Vorteilhaft sind auch Extrakte aus Pflanzen aller Art, die auf natürlich boraciferen Böden ihren Standort haben (hohe geogene Borkonzentration des Standortes).

Bei Verwendung von Extrakten wobei die Verträglichkeit der Extrakte ggf. durch Abtrennung toxischer Alkaloidkomponenten und anderer unerwünschter Begleitstoffe verbessert wird kann die Tagesdosis bei entsprechender Tolerierung das Äquivalent von 5 mg B/kg Säugetiergewicht und ggf. nach entsprechender Adaptationsperiode mehr erreichen.

Zusätzlich können neben der therapeutischen Verabreichung auch borangereicherte und natürlich borreiche Mineralwasser Verwendung finden.

Toxische Effekte bei akzidentellem Überschreiten der individuellen Bortoleranzgrenze können durch Gaben von Kupferverbindungen und in geringem Maße durch Magnesium und Calcium als Antidot neutralisiert werden.

Durch Bormanipulation in Säugetierarten gezüchtete viroid-analoge infektiöse Agentien, die sich ggf. in infiziertem Zellmaterial befinden weisen geringe oder keine Pathogenität für den vorgesehenen Wirtsorganismus auf.

Die Verwendung von durch Bormanipulation erzielten nicht oder geringfügig pathogenen subvirösen Agentien führt zum Schutz von noch nicht infizierten Geweben und Organen in von letalen Erregerstämmen befallenen Organismen, wobei dieser Schutz auf Inkompatibilitäsphänomenen und nicht auf einer klassischen Immunaktivierung (Impfantwort) beruht.

Die Erfindung wird im folgenden anhand einiger Beispiele näher erläutert.

Es wurde festgestellt, daß überraschenderweise eine Behandlung von Pflanzen und Kulturen mit bio-assimilierbaren anorganischen oder organischen wäßrigen Lösungen von Borverbindungen die Symptome der viroiden Infektion unterdrückt. Dies wurde am Beispiel des Experimentalsystems "Potato Spindle Tuber Viroid" (letaler Stamm KF 440/2) / Tomate (Sorte Rentita) festgestellt. Die borbehandelten Pflanzen entgehen ausnahmslos der zerstörerischen Wirkung durch die Viroidinfektion und führen bei permanenter und ausreichender Zufuhr z. B. von Borsäure oder neutralisiertem Borax einen normalen Lebens- und Reproduktionszyklus durch. Sie erzeugen sorgar mehr Biomasse und Früchte als die nichtinfizierten und sonst unter identischen Bedingungen aufgewachsenen

Aus WO 92/01455 ist die Verwendung von Borverbindungen zur Behandlung der senilen Demenz vom Alzheimerschen Typ bekannt, die Verwendung von Borverbindungen zur Bekämpfung subviröser Partikel ist nicht erwähnt.

Aus ACTA Virol., Bd. 34, Nr. 4, 1990, Seiten 330-338 und LATV. POSR ZINAT. AKAD. VESTIS, KIM. SER., Nr. 6, 1985, Seiten 695-702 ist die Bekämpfung von Viren durch Borverbindungen bekannt. In der FR-A-2 684 002 wird die Behandlung von erworbener Immunschwäche, die durch Retroviren verursacht wird (AIDS), mit einem Gemisch von organischen und anorganischen Verbindungen vorgeschlagen.

Kontrollpflanzen, während infizierte und nicht borbehandelte Pflanzen ausnahmslos absterben.

Die Zufuhr der Borverbindungen kann nutritiv mit dem Nährmedium oder im Boden und/oder durch foliales Auftragen erfolgen. Die Behandlung kann präventiv oder kurativ sein. Der präinfektionelle Schutz ist der wirksamste. Post-infektionell, vor allem folial aufgetragene Borverbindungen schützen die noch symptomfreien Bezirke der Pflanze und verhindern ihr Absterben, jedoch sind Störungen, die sich bereits morphologisch fixiert haben (Stauchungen, Nekrosen, Kleinfrüchtigkeit), nicht mehr rückgängig zu machen.

Hilfsweise wurde ein Teil der Pflanzen auch mit bio-assimilierbarem Silizium (neutralisiertes Wasserglas) angereichert. Dabei zeigte sich, daß Silizium die normalerweise engen Bor-Toleranzgrenzen der Pflanzen erweitert: Die toxische Wirkung der Borverbindungen wird in beträchtlich höhere Konzentrationsbereiche verschoben. Pflanzenverfügbares Silizium wirkt sozusagen als Antidot gegen die herbiziden Effekte größerer Mengen an Borverbindungen.

Dabei ist zu bemerken, daß Silizium allein ohne Bor wie alle anderen bisher getesteten Spurenelemente die Virulenz der Viroidinfektion stimuliert und das Absterben der Pflanzen beschleunigt.

Zur Sicherstellung des erfindungsgemäßen Effektes und zu seiner Abgrenzung gegen den bestehenden Stand der Technik wurde ein Reihe zusätzlicher Experimente unternommen. Es war zu bestätigen, daß es sich um einen selektiven Effekt handelt, der nicht auf einer unspezifischen, allgemeinen Erhöhung der natürlichen Widerstandskraft der Pflanze durch ein ausreichendes Angebot an dem für sie notwendigen Spurenelement Bor beruht.
1. Dies zeigt sich daran, daß andere bisher getestete Spurenelemente (z. B. Mangan, Silizium), die bekanntermaßen ebenfalls Wachstum und Widerstandskraft der Pflanze verbessern, in entgegengesetzter Richtung wirken, d. h. die pathogene Wirkung der Viroide verstärken, und daß nur Bor selektiv die beschriebene Schutzwirkung ausübt.
2. Die Tatsache, daß borgeschützte, infizierte Pflanzen mehr Biomasse erzeugen als nur borangereicherte Kontrollpflanzen ohne Viroid zeigt, daß etwas geschieht, was nicht additiv aus dem vorangehenden Kenntnisstand zusammengesetzt werden kann. Da die infizierte Pflanze zunächst die Syntheseleistung für bis zu 20 000 Viroidpartikel pro Zelle zu erbringen und dann deren pathogene Wirkung zu erleiden hat, müßte, vom bestehenden Stand der Technik ausgehend, ihre Biomassenerzeugung in jedem Fall deutlich niedriger liegen als bei den nichtinfizierter Kontrollpflanzen.
3. Die Dosierung der Viroidpartikel in dem infizierten Pflanzenmaterial durch molekulare Hybridisierung und Northern-Blot ergab für die borgeschützten Pflanzen überraschenderweise eine 5 x höhere Viroidkonzentration als in den Kontrollpflanzen mit dem normalen Borgehalt. Das Viroid ist in den Zellen der borgeschützten Pflanzen zwar vorhanden, und dazu in hoher Konzentration, aber es ist inoffensiv für die Pflanze geworden.
   Die Schutzwirkung beruht also nicht auf einer einfachen nutritiven Stärkung bereits vorhandener gegen die Viroidreplikation gerichteter pflanzlicher Abwehrkräfte, sondern auf einer Modifikation der pathogenen Eigenschaften der Parasiten. Dabei ist zu unterscheiden zwischen Infektiosität (Anzahl der infektiösen Partikel), die steigt, und Pathogenität (Ausmaß der Zellzerstörung), die neutralisiert wird. Borverbindungen bewirken die Bildung nichtpathogener Viroidstämme, die möglicherweise sogar Hilfsfunktionen im Wirtszellenmetabolismus ausüben (mehr Biomasse), was als ein Hinweis auf Herkunft und Entstehung der Viroide zu werten wäre: Für den sehr wahrscheinlichen Fall, daß Viroide evolutionär von normaler, zelleigener RNA abstammen, die außer Kontrolle geraten ist, würde die Aktion von Bor eine Umkehr des degenerativen Vorganges, d. h. einen Rücktransfert viröser RNA-Segmente in Richtung auf normale, konstruktive zelluläre Funktionen bedeuten.
4. Weiterhin war zu zeigen, daß die der Pflanze angebotenen Borverbindungen tatsächlich im infektionsbedrohten bzw. infizierten Blattmaterial am Ort der Viroidreplikation lokalisiert sind. Spektroskopische Messungen (AAS/ICP) zeigten, daß das Blattmaterial der borbehandelten Pflanzen den 10fachen Borgehalt (100 ppm) im Verhältnis zu normalen Tomatenpflanzen aufweist. Dieser verdoppelt sich nochmals bei simultaner Zugabe von Kieselsäure. Innerhalb der bereits bor-angereicherten Zellmasse ist das Bor am Ort der Viroidsynthese, d. h. im Zellkern und im Nukleolus, noch einmal selektiv konzentriert. Die Hauptmenge des der behandelten Pflanze zugeführten Bors ist also in direktem Kontakt mit dem nascierenden Viroidmolekül.
5. Das Viroid als das einfachste Modell eines Krankheitserregers besteht aus nur einem kleinen Molekül Ribonukleinsäure. Die Änderungen der Infektiosität und der Pathogenität durch die Einwirkung von Borverbindungen müssen sich in den Primär- und Sekundärstrukturen dieses alleinigen Erregermoleküls irgendwie niederschlagen und physiko-chemisch sichtbar gemacht werden können.

NMR-Messungen zeigten uns, daß Borsäure spezifisch einen Bikomplex mit dem Ribonukleotid GMP bildet und in der Lage ist, freie und 5-terminale GMP-Moleküle selektiv zusammenzuführen und so direkt in das Processing der sich bildenden Viroide einzugreifen. Mit seiner ausgeprägten Tendenz, das leere p_{z}-Orbital unter Bildung einer sp³-Tetraederstruktur aufzufüllen, übt Bor eine spezifische Affinität zu nukleophilen Liganden aus und ist in der Lage, quasi-identische Viroidmoleküle und die zugehörigen Wirtsfaktoren streng selektiv zu beeinflussen.

Bor scheint dabei die Bildung thermodynamisch stabiler Viroid-Grundkörper mit geringer Tendenz zur Schleifenbildung und einem stark verzögerten Schmelzverhalten (= intramolekulare Paarungsauflösung) zu bewirken. Stabile Viroidmodifikationen schmelzen erst bei erhöhten Temperaturen. Zur Entfaltung seiner Pathogenität und zur Erlangung eines breiten Wirtsspektrums benötigt das Viroidmolekül jedoch bereits bei Temperaturen der Infektion ausgeprägte Tertiärsturkturen mit leichter, rascher und zahlreicher Bildung von Scheifen und Verzweigungen, die die spezifische Anheftung an Faktoren der Wirtszelle ermöglichen. Die Begünstigung stabiler Viroidstämme mit erschwertem Schmelzverhalten durch das Bor erklärt zumindest teilweise seine anti-pathogene Wirkung auf die Viroide.

Die Erfindung betrifft weiterhin die Ausweitung der Methode auf die Bekämpfung mittels Borverbindungen der Pathogenität viroid-analoger infektiöser RNAs und anderer subviröser Moleküle im tierischen und menschlichen Organismus, wo diese ggf. mit Ko-Faktoren zellendogener oder -exogener Herkunft an der Auslösung der oben beschriebenen subakuten degenerativen Erkrankungen des Zentralnervensystems ursächlich beteiligt sind.

Wegen der extrem langen Reaktionszeiten und der besonders schwierigen Experimentalbedingungen wird die Wirkung von Borverbindungen hier zunächst epidemiologisch abgestützt und durch Analogieschlüsse aus den Viroidinfektionen in Pflanzen und dem physiologischen Verhalten der Borverbindungen in der Tierzelle zusätzlich belegt.

Es erweist sich, daß bei der jetzt weltweiten Verbreitung der spongiformen Encephalopathien Scrapie (Schaf) und BSE (Rind) gewisse geographische Zonen ausgespart bleiben. Es handelt sich dabei um Gegenden, die bei früheren epidemiologischen. Studien durch die Quasi-Abwesenheit tierischer und menschlicher Tuberkuloseformen (Mycobacterium tuberculosis und M. bovis) auffielen und die sich durch einen extrem hohen natürlichen Borgehalt im Boden auszeichnen, z. B. die boraciferen Vulkanregionen Italiens und Gegenden Nordwestkasachstans und des Atlasgebirges. Die dort angesiedelten Pflanzensorten haben auf dem natürlichen Bor-Standort eine erstaunliche Bortoleranz entwickelt und weisen Borgehalte auf, die für dieselbe Pflanzenart an einem durchschnittlichen Standort toxisch wären. Die hohen Borgehalte werden über die Nahrungsmittelkette in das Tierreich übertragen und nach einer Anpassungsphase von den dort lebenden Individuen toleriert. Die permanente Ingestion der borangereicherten Pflanzenteile bewirkt bei den Tieren einen Schutz gegen die Infektion bzw. gegen die Ausprägung ihrer Symptome.

In boracifere Gegenden importierte Tiere adaptieren sich zum Teil spontan anden hohen Borgehalt ihres Biotops, zum Teil wird eine Anpassungsphase benötigt; ein geringer Prozentsatz der Individuen entwickelt Symptome der Bortoxizität. Diese ähneln bei Schafen den Symptomen der Scrapie-Krankheit, sind jedoch im Gegensatz dazu bei Aufhebung des Bor-Stresses reversibel. Bortoxische Effekte können bei Tieren durch Gaben von Kupfer-, Magnesium- und Calcium- Verbindungen reduziert werden. Die erworbene Bortoleranz wird genetisch fixiert.

Als eine weitere Stütze für die Übertragbarkeit der Viroidbekämpfung durch Borverbindungen vom Pflanzen- auf den Tierbereich wurde festgestellt, daß die die Scrapie-Krankheit charakterisierenden ekzematösen Prirugos (Juckflechten) durch lokale Applikation von Borverbindungen (z. B. Boroglycerinlösung) erfolgreich behandelt werden können. Dies ist das einzige Beispiel einer, wenn auch zeitlich begrenzten, partiellen Remission bei den sonst unerbittlich progredient zum Tode führenden Infektionen.

Ein zusätzlicher Hinweis ist das zellphysiologische Verhalten der Borverbindungen. Bor ist im Pflanzen- und Tierstoffwechsel das diffusionsaktivste Element. Borate und Derivate durchdringen mit hoher Geschwindigkeit Zellen, Zellkompartimente und Organellen, hydrophile und lipophile Zonen, Organe und Organschranken (z. B. die Plazentabarriere; die Herzfasermembranen, in pathogenen Situationen öffnet sich sogar die Blut-Hirn-Schranke). Ähnlich wie die Erreger beeinflussen Borverbindungen durch Entmischung der Phospholipidstrukturen die Permeabilität der Membran (chaotropischer Effekt). Bor wird nach membranspezifischen Kriterien an- oder abgereichert.

Im infizierten tierischen und menschlichen Organismus werden die ungefähr gleichmäßig verteilten geringen Bormengen (0,5 ppm) mobilisiert und an die Stellen der nekrotischen Zellzerstörung transportiert, ein Hinweis auf die zentrale Rolle des Bors beim Überlebenskampf bzw. der apoptotischen Selbstauslöschung der infizierten Zellen.

Wegen des Nichtvorhandenseins eines geeigneten Radio- Isotopes ist die Schlüsselrolle des Bors bei der spontanen Zelldegeneration durch Autophagieprozesse bisher übersehen worden.

Durch die Sogwirkung, die die infizierten pathogenen Zonen im Organismus und auf das natürlich vorhandene oder therapeutisch applizierte Bor ausüben, wird dessen Rolle am Infektionsort kinetisch begünstigt. Wie in der infizierten Pflanze werden auch im tierischen Organismus sowohl der Erreger als auch das Bor in den gleichen Zonen konzentriert und geraten dort in optimalen Kontakt miteinander.

Wie in der Pflanze konzentriert sich das dem tierischen und menschlichen Organismus zugeführte Bor selektiv in Zonen beschleunigten Stoffwechselgeschehens, überall dort, wo Biomassen rasch entstehen oder abgebaut werden, besonders
- in durch virale oder subvirale Erreger infizierten Zellen
- in Tumor-Zellen, vor allem im Nerven- und Cerebralbereich
- in Bezirken nekrotischer Zellzerstörung (spongiforme Durchlöcherung im Hirn, nekrotische Infarkt-Bezirke in Organen)
- in der Startphase der Schwangerschaft im Fetus.

Ein weiterer Hinweis dafür, daß nicht-konventionelle Krankheitserreger und Borverbindungen spezifisch an den gleichen zellulären Schaltstellen aktiv sind, ist die Tatsache, daß die human-toxische Wirkung von hoch-überschüssiger Borsäure und die pathogene Wirkung der zu bekämpfenden sub-viralen Erreger ein fast identisches klinisches Bild erzeugen: Kopfschmerz, Depression, Konvulsion, Hyperästhesie, Tremor, Rigor, Akinese, Ataxie, Meningitis, Delirien, Sinnestäuschungen, die äußerlich von ekzematösen Prirugosbegleitet sein können.

## Patentansprüche

1. Verwendung von wäßrigen Lösungen von Borverbindungen zur Herstellung eines Arzneimittels zur Bekämpfung subviröser Partikel, die im Tier- und Humanbereich subakute, degenerative, nicht inflammatorische Erkrankungen des Zentralnervensystems, mit Ausnahme von Krankheiten vom Alzheimer Typ, hervorrufen oder zum Schutz gegen die Wirkung subviröser Krankheitserreger in Pflanzen.

2. Verwendung von wäßrigen Lösungen von Borverbindungen nach Anspruch 1 zum Schutz gegen die Wirkung subviröser Krankheitserreger in Pflanzen präventiv und/oder kurativ.

3. Verwendung von wäßrigen Lösungen von Borverbindungen nach Anspruch 1 oder 2, wobei die Dosierung der Borverbindungen der zu schützenden Pflanzenspezies und -sorte angepaßt ist.

4. Verwendung von wäßrigen Lösungen von Borverbindungen nach Anspruch 3, wobei die Pflanze bis zur oberen Toleranzgrenze borangereichert wird.

5. Verwendung von wäßrigen Lösungen von Borverbindungen nach Anspruch 4, **dadurch gekennzeichnet, daß** die obere Toleranzgrenze für Bor durch simultane Applikation von löslichen Silikaten in beträchtlich höhere Konzentrationsbereiche verschoben wird.

6. Verwendung von wäßrigen Lösungen von Borverbindungen nach einem der Ansprüche 1 bis 5, zur Züchtung nichtpathogener, inoffensiver Viroidstämme in hochborangereicherten Pflanzen, wobei die Viroidstämme ein starkes Proliferationsvermögen besitzen, d. h. hohe Infektiosität bei geringer oder ohne Toxizität aufweisen.

7. Verwendung der Viroidstämme nach Anspruch 6 zur präventiven Beimpfung gefährdeter Kulturen als Infektionsschutz gegen verwandte Viroidstämme hoher Pathogenität.

8. Verwendung von wäßrigen Lösungen von Borverbindungen nach Anspruch 1, wobei die Borverbindungen zur Herstellung des Arzneimittels Extrakte aus borangereicherten Pflanzen sind.

9. Verwendung von wäßrigen Lösungen von Borverbindungen nach Anspruch 8, **dadurch gekennzeichnet, daß** aus den Extrakten toxische Alkaloidkomponenten und/oder andere unerwünschte Begleitstoffe abgetrennt werden.

## Claims

1. Use of aqueous solutions of boron compounds for the production of a drug for combatting sub-viral particles which cause, in animals and humans, sub-acute, degenerative, non-inflammatory diseases of the central nervous system, with the exception of diseases of the Alzheimer type, or to protect against the effect of sub-viral causes of disease in plants.

2. Use of aqueous solutions of boron compounds according to claim 1 for protection against the effect of sub-viral causes of disease in plants preventatively and/or curatively.

3. Use of aqueous solutions of boron compounds according to claim 1 or 2, wherein the dosage of the boron compounds is adapted to the plant species and plant type to be protected.

4. Use of aqueous solutions of boron compounds according to claim 3, wherein the plant is enriched with boron up to the upper tolerance limit.

5. Use of aqueous solutions of boron compounds according to claim 4, **characterised in that** the upper tolerance limit for boron is displaced by the simultaneous application of soluble silicates in considerably higher concentration ranges.

6. Use of aqueous solutions of boron compounds according to one of claims 1 to 5, for the cultivation of non-pathogenic, inoffensive viroid stems in plants highly enriched with boron, wherein the viroid stems have a strong proliferation capability, i.e. they have a high degree of infectivity with little or no toxicity.

7. Use of the viroid stems according to claim 6 for the preventative inoculation of endangered cultures to guard against infection caused by related viroid stems of high pathogenity.

8. Use of aqueous solutions of boron compounds according to claim 1, wherein the boron compounds are extracts from boron-enriched plants to produce the medicine.

9. Use of aqueous solutions of boron compounds according to claim 8, **characterised in that** toxic alkaloid components and/or other undesirable accompanying substances are separated from the extracts.

## Revendications

1. Utilisation de solutions aqueuses de composés du bore en vue de la production d'un médicament pour la lutte contre les particules subvirales qui provoquent dans le domaine animal et humain des maladies suborifiées, dégénératives, non inflammatoires du système nerveux central, à l'exception de maladies du type Alzheimer ou pour la protection contre l'action d'agents pathogènes subviraux dans les plantes.

2. Utilisation de solutions aqueuses de composés du bore selon la revendication 1,
en vue de la production contre l'action d'agents pathogènes subviraux, d'une manière préventive et/ou curative dans les plantes.

3. Utilisation de solutions aqueuses de composés du bore selon la revendication 1 ou la revendication 2,
dans laquelle le dosage des composés du bore est adapté à l'espèce ou à la sorte de plante à protéger.

4. Utilisation de solutions aqueuses de composés du bore selon la revendication 3,
dans laquelle les plantes sont enrichies en bore jusqu'à la limite supérieure de tolérance.

5. Utilisation de solutions aqueuses de composés du bore selon la revendication 4,
**caractérisée en ce que**
la limite supérieure de tolérance pour le bore est déplacée par application simultanée de silicates solubles, à des domaines de concentration notablement plus élevés.

6. Utilisation de solutions aqueuses de composés du bore, selon l'une des revendications 1 à 5,
en vue de la culture de souches de viroïdes non pathogènes, inoffensifs, dans des plantes hautement enrichies en bore, dans laquelle les souches de viroïdes possèdent une forte capacité de prolifération c'est-à-dire manifestent une infectiosité élevée pour une toxicité réduite ou sans toxicité.

7. Utilisation des souches de viroïdes selon la revendication 6,
pour l'inoculation préventive de cultures menacées, en tant que protection contre l'infection contre des souches de viroïdes apparentées de haute pathogénicité.

8. Utilisation de solutions aqueuses de composés du bore selon la revendication 1,
dans laquelle les composés du bore en vue de la production du médicament sont des extraits de plantes enrichies en bore.

9. Utilisation de solutions aqueuses de composés du bore selon la revendication 8,
**caractérisée en ce que**
les composants d'alcaloïdes toxiques et/ou d'autres impuretés non désirées sont séparés des extraits.
